# EUROPÄISCHE PATENTSCHRIFT

(11) **EP 1 558 877 B1**
(45) Veröffentlichungstag und Bekanntmachung des Hinweises auf die Patenterteilung: **12.08.2009**
(21) Anmeldenummer: 03809753.1
(22) Anmeldetag: 31.10.2003
(51) Int. Cl.: F24F 6/02, A61M 16/10

(54) **VORRICHTUNG UND VERFAHREN ZUR TEMPERIERUNG UND BEFEUCHTUNG VON GAS, INSBESONDERE VON ATEMLUFT**
DEVICE AND METHOD FOR TEMPERING AND HUMIDIFYING GAS, ESPECIALLY RESPIRATORY AIR
DISPOSITIF ET PROCEDE D'EQUILIBRAGE DE LA TEMPERATURE ET D'HUMIDIFICATION DE GAZ, NOTAMMENT D'AIR RESPIRATOIRE

(30) Priorität: 31.10.2002 DE 10251134
(43) Veröffentlichungstag der Anmeldung: 03.08.2005
(73) Patentinhaber: Gründler GmbH, 72250 Freuenstadt (DE)
(72) Erfinder: GRÜNDLER, Christoph, 72250 Freudenstadt (DE); GRÜNDLER, Markus, 72250 Freudenstadt (DE); HEINE, Daniel, 72280 Dornstetten (DE)
(74) Vertreter: Lemcke, Brommer & Partner
(86) Internationale Anmeldenummer: PCT/EP2003/012140
(87) Internationale Veröffentlichungsnummer: WO 2004/040202

(56) Entgegenhaltungen:
- DE-A- 10 049 869
- US-A- 2 162 462
- US-A- 3 987 133
- US-A- 6 068 609

## Beschreibung

Die Erfindung betrifft eine Vorrichtung zur Temperierung und Befeuchtung von Gas, insbesondere von Atemluft, wobei die Vorrichtung ein Plüssigkeitsreservoir aufweist, sowie eine Befeuchtungskammer, die einen Zufluss und einen Abfluss für das Gas aufweist, und eine Bewegungsvorrichtung, um die Flüssigkeit durch das Gas zu bewegen. Außerdem betrifft die Erfindung ein entsprechendes Verfahren.

Eine solche Vorrichtung soll insbesondere benutzt werden für die Beatmung eines Patienten. Dabei wird im Rahmen der hier beschriebenen Erfindung unter dem Begriff "Beatmen" auch jegliche Art von Atemtherapie verstanden und unter "Patienten" sind sowohl Menschen als auch Tiere zu verstehen.

Ein derartiges Beatmen eines Patienten wird üblicherweise mit Hilfe eines Apparates durchgeführt, der einen Beatmungsschlauch aufweist, ein Atemstromerzeugungsgerät und einen Befeuchter für das Atemgas..Dem Atemstromerzeugungsgerät wird das Atemgas aus einem Reservoir zugeführt und es weist auch einen Anschluss für den Beatmungsschlauch auf.

Unter einem Atemstromerzeugungsgerät ist dabei im Wesentlichen eine Funktionseinheit zu verstehen, die das einem Patienten zu applizierende Atemgas druck- und/oder volumenstrommäßig reguliert, beispielsweise mit Ventilen oder einem Blasebalg. Diese Funktionseinheit kann aber auch Teil einer größeren Apparatur sein.

Bei dem Atemgas wird es sich üblicherweise um Luft/Sauerstoff-Mischungen handeln, es können aber auch spezielle andere Gasmischungen verabreicht werden.

Bei der Flüssigkeit, die im Flüssigkeitsreservoir vorhanden ist und mit der die Befeuchtung des Gases bzw. der Luft erfolgt, handelt es sich üblicherweise um Wasser. Im Rahmen der vörliegenden Erfindung sind hierbei aber auch andere Flüssigkeiten oder Mischungen aus Flüssigkeiten denkbar, sowie auch solche, denen Medikamente zugesetzt worden sind.

Patienten, die durch Geräte beatmet werden, wird zumeist ein Tubus in die Luftröhre eingeführt, über den die Beatmung erfolgt. Dieser Tubus sitzt dabei an dem Ende eines Beatmungsschlauches, durch den hindurch ein Atemstromerzeugungsgerät Atemgas an den Patienten abgibt. Dabei können am Tubus auch weitere Beatmungsschläuche enden.

Die Abgabe von Atemgas über das Atemstromerzeugungsgerät erfolgt üblicherweise in Abhängigkeit von unterschiedlichen Parametern, die an dem Atemstromerzeugungsgerät bedarfsgerecht eingestellt werden können und dann durch dieses Gerät automatisch gehalten oder aber bedarfsgerecht variiert werden können. Je nach Gerät bzw. eingestellten Parametern ist der Atemstrom im zeitlichen Verlauf der Beatmungsphase sehr unterschiedlich. Bei bisher bekannten Beatmungssystemen führt dies zu erheblichen Problemen:

Durch den Tubus in der Luftröhre wird nämlich die natürliche Funktion des Nasen-, Mund- und Rachenraumes umgangen, die bei normaler Atmung das Atemgas erwärmt und befeuchtet. Auch bei der Atemtherapie, wie z. B. der kontinuierlichen Überdruckatemtherapie mit Gesichtsmaske (CPAP), bleibt zwar der Nasen-Rachenraum vom Atemgas durchströmt, durch den im Vergleich zur natürlichen Eigenatmung eines Patienten aber wesentlich höheren und häufig auch kontinuierlichen Gasfluss treten aber insbesondere bei Anwendung trockener kalter Gase häufig unerwünschte Nebenwirkungen auf. Hierzu gehören Irritationen und Entzündungen sowie Austrocknungen und Verborkungen der oberen Luftwege.

Um dieses Problem zu überwinden, wird bei der Beatmung das verwandte Gas, das aus einem Reservoir wie einer Gasflasche oder einer Druckgasleitung stammt oder aber auch durch ein Gebläse, einen Blasebalg oder ähnliches aus der Umgebung als Reservoir entnommen wird und das üblicherweise relativ trocken ist, künstlich befeuchtet und erwärmt.

Man versucht dabei, soweit wie möglich die natürlichen Verhältnisse zu erreichen, also das Atemgas auf eine Temperatur entsprechend der Körpertemperatur zu temperieren und es zu befeuchten, so dass es weitgehend gesättigt ist. Angestrebt wird dabei insbesondere eine relative Feuchte von ca. 95-100 %.

Dies ist besonders schwierig bei den oben beschriebenen stark schwankenden Gasströmen, wie sie bei der Spontanatmung bzw. der künstlichen Beatmung auftreten.

Eine ähnliche Problematik findet sich auch in anderen medizinischen Bereichen, in denen eine erfindungsgemäße Vorrichtung zum Einsatz kommen kann, wie z. B. in der Laparoskopie. Bei dieser wird Gas (häufig Kohlendioxid) zum Zwecke der Aufdehnung von Körperhöhlen in den Körper, z. B. in den Bauchraum, eingebracht. Auch bei dieser und ähnlichen Anwendungen kann durch eine Temperierung und Befeuchtung vorzugsweise bis zu einer weitgehenden Sättigung des verwandten Gases eine bisher häufig austretende Irritation von Schleimhäuten sowie deren Austrocknet und Auskühlen verhindert werden.

Hierbei ist zu berücksichtigen, dass auch bei dieser Anwendung stark schwankende Gasströme festzustellen sind, da das Einbringen oder Entfernen von Instrumenten in oder aus Körperöffnungen eine schnelle Nachregelung des Gasstromes erforderlich macht, um den Druck in einer Körperhöhle konstant zu hatten.

Grundsätzlich sind verschiedene Techniken und Verfahren bekannt, um Gas in oben genannten Anwendungen auf einen Vorgabewert zu temperieren und zu befeuchten. Im Folgenden sollen einige Verfahren und Vorrichtungen hierfür beschrieben werden:
Pass-over-Verdampfer (z. B. DE 38 30 314)

Bei dieser vorbekannten Vorrichtung wird ein zu temperierendes und zu befeuchtendes Gas über die Oberfläche eines beheizten Wasserbades geleitet und dabei temperiert und gleichzeitig befeuchtet. Die Flüssigkeitsoberfläche kühlt dabei in Folge der Wasserverdunstung ab und wird erst langsam durch wärmeres, nach oben steigendes Wasser ausgetauscht.

Aufgrund der durch die Anwendung in einem Beatmungssystem beschränkten Baugröße ist die Austauschfläche zwischen Flüssigkeit und darüber hinwegströmendem Gas sehr klein. Auch die beschriebene Abkühlung der Oberfläche und der daraus resultierende nur langsame Energietransport durch wärmeres Wasser an die Oberfläche führt bei einem derartigen Befeuchter dazu, dass ein austretendes Gas je nach Gasstrommenge kleinere oder größere Temperaturdifferenzen zur Flüssigkeit aufweist. Deshalb wird bei einer Vorrichtung dieser Art die Temperatur der Flüssigkeit auch nur auf eine durchschnittliche Gasstrommenge eingeregelt und bewegt sich bei herkömmlichen Geräten zwischen ca. 40 °C und ca. 80 °C. Stark schwankende Gasströme, wie sie gemäß den obigen Ausführungen in der Beatmung normal sind, haben somit in der Momentanwertbetrachtung entweder eine zu hohe oder aber eine zu niedrige Temperatur und auch eine zu hohe oder eine zu niedrige Feuchte. Dies ließe sich nur durch eine nur theoretisch mögliche schlagartige Änderung der Wassertemperatur nachregeln.

### Membran-Verdampfer (z. B . DE 43 03 645 oder ähnlich US 6068609)

Bei einer derartigen Vorrichtung wird Gas über die Oberfläche eines aus temperierter Flüssigkeit herausragenden Strukturkörpers geleitet. Der Strukturkörper saugt dabei die benötigte Flüssigkeit z. B. mittels Kapillarkräften an. Dabei wird nur die jeweils verdunstete Flüssigkeit durch neue ersetzt. Nachteilig ist hier insbesondere, dass durch die entstehende Verdunstungskälte und den nur geringen Energienachschub durch wärmeres Wasser ein ähnliches Problem wie bei den zuvor diskutierten Pass-over-Verdampfern entsteht, so dass letztlich ein variabler Gasfluss weder konstant befeuchtet noch konstant temperiert werden kann. Nur am Rande sei erwähnt, dass die angesprochenen Strukturkörper häufig nur bedingt haltbar sind und auch ihre für den medizinischen Einsatz wünschenswerte Autoklavierbarkeit meist eingeschränkt ist.

### Hohlfaser-Befeuchter (z. B. DE 197 27 884)

Teildurchlässige Hohlfasern (z. B. aus PTFE) werden bündelweise so angeordnet, dass ein zu temperierendes und zu befeuchtendes Gas z. B. durch die Lumen der Hohlfasern strömt, auf deren Außenseiten die für die Befeuchtung benötigte Flüssigkeit vorhanden ist. Nachteilig an diesen Befeuchtern ist sowohl die häufig nur eingeschränkte Haltbarkeit als auch die eingeschränkte mechanische und thermische Belastbarkeit der Hohlfasern. Außerdem ist durch die Wand der Hohlfasern keine ausreichende Wärmeleitfähigkeit gegeben, so dass der Wärmenachschub zum Ausgleich der Verdunstungskälte nicht ausreicht. Somit ist insbesondere bei höheren Gasströmen eine erwünschte Erwärmung des Gases nicht zu gewährleisten, was gleichzeitig mit einer mangelhaften Befeuchtung des Gases einhergeht. Auch hier kann theoretisch durch eine Erwärmung des Wassers nachgeregelt werden, allerdings führt auch die zwangsweise Erwärmung der Fasern bei stark schwankendem Gasstrom nicht zu einer konstanten Befeuchtung, da die Energieabgabe der Erwärmungselemente aus technischen Gründen kaum in der Geschwindigkeit der Gasstromänderung nachregelbar ist.

### Hochtemperaturverdampfer (z. B. DE 43 12 793 oder US 2162 462)

Bei derartigen Vorrichtungen werden bei Temperaturen von ca. 80 °C bis ca. 130 °C kleine Flüssigkeitsmengen verdampft und mit dem strömenden Gas vermischt. Hierdurch wird sowohl die benötigte Energie zur Temperierung des Gases als auch die benötigte Feuchte bereitgestellt. Nachteilig bei diesen Vorrichtungen ist insbesondere der hohe technische Aufwand, der außerdern noch mit einem erhöhten technischen Risiko verbunden ist, insbesondere im Hinblick auf Gefahren durch Druck und Hitze. Des weiteren haben die Vorrichtungen den Nachteil, dass die Regelung der benötigten Verdampfungsmenge nur mit zeitlicher Verzögerung erfolgen kann. Damit ist auch mit einer derartigen Vorrichtung bei stark schwankendem Gasstrom keine konstante Temperierung und Befeuchtung zu erreichen.

### Durchsprudel-Befeuchter (z. B. DE 37 30 551 oder US 3 987 133)

Bei Vorrichtungen dieser Art wird durch eine temperierte Flüssigkeit Gas (hindurch) gesprudelt, wodurch es befeuchtet und temperiert wird. Der Nachteil dieses Verfahrens ist insbesondere der verfahrensbedingte hohe Gasströmungswiderstand, da stets mindestens ein Differenzdruck zu überwinden ist, der der Eintrittstiefe des Gases in der Flüssigkeit entspricht. Dies ist vor allem bei spontan atmenden Patienten ausgesprochen nachteilig.

### Ultraschallvernebler (z. B. DE 197 26 110)

Bei derartigen Vorrichtungen wird Flüssigkeit zu Schwingungen im Ultraschallbereich angeregt, was dazu führt, dass sich kleinste Tröpfchen von der Flüssigkeit lösen und mit dem Gasstrom mitgerissen werden können. Hierbei ist insbesondere nachteilig, dass es sich keinesfalls um in molekularer Form vorliegende Flüssigkeit handelt sondern um wesentlich größere Einheiten (Aerosole). Hierdurch können verschiedenste Krankheitserreger in unerwünschter Weise transportiert werden. Des weiteren besteht bei diesem Verfahren auch die Gefahr, dass zu viel oder zu wenig Feuchte abgegeben wird, insbesondere bei intermittierenden oder schwankenden Gasströmen.

### Druckzerstäuber (z. B. DE 28 34 622)

Bei Vorrichtungen dieser Art wird eine Flüssigkeit verdüst, was dazu führt, dass sich ebenfalls kleinste Tröpfchen bilden und nicht z. B. molekularer Wasserdampf entsteht. Die Nachteile dieser Druckzerstäuber entsprechen somit den zuvor diskutierten Ultraschallverneblem.

### Künstliche Nasen ("heat and moisture exchanger"; HME, z. B. DE 94 17 169), Filtermatten, etc.

Bei künstlichen Nasen wird das Gas über eine sehr große angefeuchtete Oberfläche geleitet, wodurch im Wesentlichen eine Feuchtigkeitssättigung des Gasstromes erreicht wird. Bei den künstlichen Nasen erfolgt die Wärme- und Feuchtigkeitszufuhr über die Ausatemluft eines Patienten. Bei Filtermatten, z. B. aus der Klimatechnik, erfolgen Wärme- und Feuchtezufuhr z. B. über ein Wasserbad. Über diese Filtermatten wird gleichzeitig eine Filtration des Gases durchgeführt.

Nachteilig ist bei diesen bekannten Vorrichtungen insbesondere, dass die Verdunstungskälte zu einer gasstromabhängigen Abkühlung führt, so dass Befeuchtung und Temperierungsleistung bei variablem Gasstrom nicht konstant sein können. Des weiteren ist bei diesen Vorrichtungen nachteilig, dass die bei der Filtration zurückgehaltenen Stoffe im Laufe der Einsatzdauer den Durchflusswiderstand der Filtermatten für das Gas erhöhen, was speziell im medizinischen Bericht unerwünscht ist. Auch wird durch die prinzipbedingte Anordnung der HMEs im Ein- und Ausatembereich der Patienten der Totraum nachteilig vergrößert. Dies kann dazu führen, dass ein Patient bei einem Atemzug einen Großteil der Luft einatmet, die er zuvor gerade ausgeatmet hat.

### Booster-Systeme (z. B. DE 44 32 907)

Bei diesen Systemen wird versucht, den unzureichenden Wirkungsgrad von künstlichen Nasen (HME) mittels Zufuhr von Flüssigkeit und Wärme auszugleichen. Dies erfordert eine technisch recht aufwändige Regelung. Außerdem können systembedingt auch hier bei stark schwankendem Gasstrom Temperatur und Feuchte nicht konstant gehalten werden, da auch eine sehr gute Regelung die entstehenden Verdunstungskälte kaum ohne Zeitverzögerung ausgleichen kann. Der oben beschriebene nachteilige Totraum, sowie die nachteilige Baugröße und das nachteilige Gewicht werden bei diesen Systemen durch weitere, hier nicht näher erläuterte Elemente weiter vergrößert.

### Kombination von bisher diskutierten Verfahren (DE 296 12 115).

Bei diesem kombinierten Verfahren wird das Gas zunächst überhitzt und befeuchtet und in einer nachfolgenden zweiten Stufe dann mittels konstant temperierter Metallrippen o. ä. auf die Zieltemperatur gekühlt. Überschüssige Feuchte im Gas wird hierbei als Kondensat von den temperierten Metallrippen tropfen und wieder dem Befeuchter zugeführt. Nachteilig bei diesem Verfahren ist vor allem, dass dem Gas zuerst mehr Energie in Form von Feuchte und Temperatur zugeführt wird, als für die Beatmung notwendig ist.

Abgesehen davon, dass dies energetisch ungünstig ist, besteht auch die Gefahr, dass bei einer Fehlfunktion in der Abkühlstufe ein Patient dann nachhaltig geschädigt werden kann.

### Raumluft-Befeuchter mit rotierendem Plattenstapel (z. B. DE 37 35 219)

Bei derartigen Systemen rotieren Plattenstapel derart, dass die Platten bei einem Teil ihres Umlaufes in Wasser eintauchen und dadurch benetzt werden. Das Gas strömt mittels eines Ventilators an diesen Plattenstapeln vorbei und soll somit von Partikeln gereinigt und befeuchtet werden. Bei diesen Vorrichtungen soll der Flüssigkeit ein nicht flüchtiges Mittel zugesetzt werden zur Reduktion der Oberflächenspannung, um tatsächlich eine ausreichende Plattenbenetzung zu erreichen. Diese und ähnliche Vorrichtungen werden insbesondere für die Wohnraum-Klimatisierung vorgesehen und weisen keine Möglichkeit zur Temperierung des Gases auf. Außerdem ist die Dimensionierung und die Umlaufgeschwindigkeit der Platten in keinster Weise geeignet, um eine variable Gasströmung konstant zu befeuchten oder gar mit einer Flüssigkeit zu sättigen.

Es muss zusammenfassend also festgestellt werden, dass im Stand der Technik keine Verfahren und Systeme für die Temperierung und Befeuchtung von Gasen vorhanden sind, die für die Anwendung bei stark schwankender oder intermittierender Gasströmung geeignet wären. Bei den bisher bekannten Vorrichtungen kommt es dabei zu erheblichen Schwankungen von Temperatur und Feuchte des abgegebenen Gases.

Ein weiterer Nachteil der bekannten Geräte besteht darin, dass sie die Präzision von Mess- und Regelvorgängen, welche für eine Beatmung gewünscht werden, teilweise erheblich verschlechtern oder sogar ganz verhindern: Beispielsweise gibt es Methoden und Sensoriken zur Beatmung, die eine möglichst unmittelbare Ankopplung eines Atemstromerzeugungsgerätes bzw. eines Sensors an einen Patienten benötigen, um diesem das benötigte Atemgas in einer vorgegebenen konstanten Qualität und Quantität und insbesondere auch mit präzisen Volumenströmen zu verabreichen.

Die heute bekannten Befeuchter, die zwischen einem Atemstromerzeugungsgerät und dem Patienten angeordnet sind, sind insofern nachteilig als mit ihnen ein zusätzliches kompressibles Volumen von teilweise erheblicher Größe in den Atemkreislauf eingebracht wird.

Ein weiterer Nachteil der bekannten Befeuchter ist auch der Druckgradient, der bei manchen herkömmlichen Befeuchtem zwischen deren Eingang und Ausgang herrscht: Da der Druck des dem Patienten zugeführten Atemgases (Atemgasniveau) nur gering höher ist als der Umgebungsdruck (üblicherweise maximal ca. 0,1 bar) führt dieser Druckgradient dazu, dass am Atemstromerzeugungsgerät selbst nicht exakt der am Patienten herrschende Druck anliegt. Dies hat die Gefahr von Fehlfunktionen und Ungenauigkeiten in der Regelung als Konsequenz.

Aufgabe der vorliegenden Erfindung ist es daher, Vorrichtungen wie oben angegeben derart weiterzubilden, dass die genannten Nachteile beseitigt werden und insbesondere Gas unabhängig von Gasstromschwankungen konstant auf einen Vorgabewert zu temperieren ist und es insbesondere bei dieser Temperatur mit einer Flüssigkeit weitgehend zu sättigen ist, das heißt bis zu einer relativen Feuchte von ca. 95 - 100 %.

Diese Aufgabe wird erfindungsgemäß dadurch gelöst, dass eine Vorrichtung zur Temperierung und Befeuchtung von Gas mit einem Flüssigkeitsreservoir sowie einer Befeuchtungskammer, die einen Zufluss und einen Abfluss für das Gas aufweist, sowie mit einer Bewegungsvorrichtung; die dazu angepasst ist um die Flüssigkeit durch das Gas zu bewegen, und eine geregelte Heizung aufweist, die dazu angepasst ist, die Flüssigkeit zu beheizen.

Die Erfindung hat gegenüber dem Stand der Technik mehrere Vorteile:

Zum einen besteht die Möglichkeit, durch die Bewegungsvorrichtung und die geregelte Heizung zwei Elemente zu haben, über die eine Regelung der Temperierung und Befeuchtung zu erreichen ist.

Zum anderen kann über diese zwei Elemente, die unabhängig voneinander zu regeln sind, eine recht intensive Temperierung und Befeuchtung erreicht werden, so dass die Vorrichtung relativ kompakt ausgebildet werden kann.

Des weiteren ist eine derartige Vorrichtung robust und betriebssicher ausführbar und ist damit auch im Dauerbetrieb zuverlässig einzusetzen.

Es werden auch keine Aerosole erzeugt sondern das Gas wird mit dampfförmiger Flüssigkeit gesättigt. Wesentlich ist auch, dass die Regelung der Temperierung und Befeuchtung über die Bewegungsvorrichtung und die geregelte Heizung einfach nachzuregeln ist.

Vorteilhafterweise wird dabei die Befeuchtungskammer als Berieselungskammer ausgebildet. In einer Berieselungskammer wird die Flüssigkeit durch das Gas hindurchbewegt. Die Temperatur des durchströmenden Gases kann sich dabei leicht an die Temperatur des Wassers annähern, wobei sich gleichzeitig eine Sättigung des durchströmenden Gases mit verdunstender Flüssigkeit ergibt. Das aus der Berieselungskammer austretende Gas ist feuchtigkeitsgesättigt, d. h. dass es eine relative Feuchte von ca. 95 -100 % aufweist und hat eine Temperatur nahe der vorgegebenen Wassertemperatur.

Vorteilhafterweise ist in der Berieselungskammer auch eine Füllung vorgesehen mit einer großen Oberfläche. Die Füllung wird durch die ständig über sie hinweglaufende Flüssigkeit auf die gleiche Temperatur gebracht, die die Flüssigkeit hat. Die Füllung kann somit als Puffer dienen, um bei stark schwankenden Gasströmen und sich damit stark ändernden Bedürfnissen an Verdunstungsenergie kurzfristig Energie wieder für einen erhöhten Bedarf an Verdunstungsenergie zur Verfügung stellen zu können.

Als besonders geeignet hat sich für diese Füllung daher z. B. ein Aluminiumgestricke oder aber Edelstahlwolle gezeigt, als auch eine Packung aus Metall-, insbesondere Stahlkugeln. Diese Materialien haben eine hohe Energiespeicherkapazität kombiniert mit der Eigenschaft, Energie auch schnell wieder abgeben zu können.

Es sind aber auch andere offenporige Strukturen für das Füllungsmaterial denkbar.

Wesentlich ist, dass die Füllung so gewählt wird, dass der Volumenstrom des Atemgases strömungstechnisch nicht wesentlich behindert wird und somit auch bei einem maximalen Volumenstrom kaum eine merkliche Druckdifferenz zwischen Zufluss und Abfluss des Gases festzustellen ist.

Eine solche Füllung ist bei einer weiter bevorzugten Ausführungsform auch einfach aus der Berieselungskammer zu entnehmen zum Zwecke des Auswechselns oder zur Sterilisierung.

Weiterhin ist wesentlich, dass die Bewegungsvorrichtung, um die Flüssigkeit durch das Gas zu bewegen, bei der es sich vorzugsweise um eine Pumpe handelt, die in ihrer Temperatur geregelte Flüssigkeit in einer Menge durch das Gas und damit gegebenenfalls über eine Füllung in der Berieselungskammer führt, die so groß ist, dass sie die Energie bereitstellen kann, sowohl um die Temperatur des durchströmenden Gases anzuheben, als auch um die benötigte Verdunstungsenergie im Wesentlichen bereitzustellen. So ist zu erreichen, dass bei maximalem Volumenstrom über das Berieselungselement das durchströmende Gas auf nahezu Flüssigkeitstemperatur erwärmt wird und mit einer relativen Feuchte von nahezu 100 % gesättigt abfließt.

Es hat sich als vorteilhaft erwiesen, die Befeuchtungskammer in bestimmten Anwendungen unter Überdruck zu stellen.

Damit hat man die Möglichkeit, einem nachgeschalteten Atemstromerzeugungsgerät bereits angefeuchtete Luft zuführen zu können und eine Befeuchtung muss nicht erst zwischen dem Atemstromerzeugungsgerät und dem Patienten erfolgen. Hierdurch wird nicht nur Totraum (komprimierbares Volumen) im Bereich zwischen Atemstromerzeugungsgerät und Patient vermieden, sondern auch ein Einbau der Vorrichtung im Beatmungsschlauch, der zwischen Atemstromerzeugungsgerät und Patient verläuft, was dort einen zusätzlichen unerwünschten Druckgradienten bewirken könnte.

Außerdem kann durch die Befeuchtung unter Überdruck in der Vorrichtung eine wesentlich größere Gasmenge befeuchtet und auch bevorratet werden, als bei einem Befeuchter, der im Bereich niedrigeren Drucks zwischen Atemstrom-Erzeugungsgerät und Patient angeordnet ist. Dies bewirkt eine als positiv anzusehende Pufferung von erwärmtem und befeuchtetem Gas und somit kann ein Ausgleich bei kurzfristig schwankenden Volumenströmen erfolgen.

Eine aufwändige Regelung ist nicht erforderlich. Dies bedeutet auch eine erhebliche konstruktive Vereinfachung, die zu Kosteneinsparungen führt.

Um dabei vom Atemstromerzeugungsgerät zum Patienten strömendes Gas präzise in der Temperatur auf den benötigten Wert einzustellen, wird weiterhin vorgeschlagen, den Beatmungsschlauch selbst mit einem entsprechenden Heizelement zu versehen, das zum Beispiel im Schlauchinneren platziert ist. Es ist aber auch möglich, das Heizelement insbesondere in die Wandung des Beatmungsschlauches einzuarbeiten. Die Integrierung des Heizelementes in den Beatmungsschlauch hat den Vorteil, dass das vorgesehene Heizelement im direkten Kontakt mit dem Atemgas ist und direkt auf die gewünschte Endtemperatur eingestellt werden kann, womit es erleichtert wird, bei stark schwankenden Volumenströmen immer eine gleichmäßige Temperatur des Gases am Patienten zu haben.

Es sei noch erwähnt, dass bei einer mit Überdruck beaufschlagten Befeuchtungskammer die Flüssigkeit und damit auch der Befeuchtungsprozess auf eine Temperatur eingestellt wird, die so gewählt ist, dass auch nach der nachfolgenden Entspannung des mit Feuchtigkeit angereicherten und erwärmten Gases auf Beatmungs- bzw. Umgebungsdruck und bei einer gewünschten Temperatur (Beatmungstemperatur) eine definierte Luftfeuchtigkeit erreicht wird. Diese liegt in Beatmungsfällen vorzugsweise ebenfalls nahe der Sättigungsgrenze.

Grundsätzlich ist die Temperatur in der unter Überdruck stehenden Befeuchtungskammer dabei sowohl abhängig von dem im Befeuchter herrschenden Druck als auch von der patientenseitig gewünschten Temperatur und der patientenseitig gewünschten relativen Feuchte eines Atemgases. Je nach Anwendungsfall können dabei am Patienten auch geringere relative Feuchten gewünscht sein als 100 %.

Es liegt dabei auch im Rahmen der Erfindung, bei schwankenden Gasströmen den Überdruck den Schwankungen entsprechend zu variieren, um damit auf der Patientenseite ein nach der Entspannung konstant temperiertes und befeuchtetes Atemgas anzubieten.

Für die Flüssigkeit, die nicht für die Befeuchtung des Atemgases selbst benötigt wird, sondern lediglich zu dessen Erwärmung dient und die somit in dem Berieselungselement an Temperatur verliert, wird bei einer bevorzugten Ausführungsform vorgesehen, sie zum Flüssigkeitsreservoir zurückzuführen. Von dort kann sie nach einer erneuten Erwärmung über die geregelte Heizung wieder dem Berieselungselement zugeführt werden.

In einen derart gebildeten Kreislauf wird dabei vorzugsweise ein Filter integriert, durch den gewährleistet wird, dass sowohl die umlaufende Flüssigkeit als auch das Atemgas im Wesentlichen keimfrei gehalten werden.

Bei einer bevorzugten Ausführungsform ist das Flüssigkeitsreservoir, das auch die geregelte Heizung für die Flüssigkeit aufweist, drucklos und ist über eine Pumpe (als Bewegungsvorrichtung für die Flüssigkeit) mit dem Berieselungselement verschaltet. Indem diese Pumpe geregelt wird, kann die Menge der umgewälzte Flüssigkeit an den entsprechenden Bedarf angepasst werden. Indem im Umwälzkreislauf gegebenenfalls noch ein Druckminderer vorgesehen ist, kann auch bei einer unter Überdruck stehenden Befeuchtungskammer ein unterbrechungsfreier Betrieb der Temperierungs- und Befeuchtungsvorrichtung möglich sein, während eines notwendigen Nachfüllens von Flüssigkeit in das Flüssigkeitsreservoir.

Natürlich kann der beschriebene Berieselungsbefeuchter auch im Niederdruckbereich eingesetzt werden. Die skizzierten Vorteile bleiben dabei erhalten. Es kann allerdings sein, dass sich bei Integrierung in ein Atemgerät dadurch unerwünschte Toträume ergeben.

Eine alternative Ausführungsform weist als Bewegungsvorrichtung für die Flüssigkeit keine Pumpe auf, sondern einen in die Flüssigkeit in dem Flüssigkeitsreservoir teilweise eintauchenden rotierenden Körper. Bei diesem handelt es sich vorzugsweise um einen Stapel zueinander beabstandeter Platten, die um eine horizontale Achse rotieren und dabei aus dem Flüssigkeitsreservoir Flüssigkeit in den Gasstrom schleppen, wo die Flüssigkeit dann verdunstet und zu einer Befeuchtung des Gases führt. Eine solche Vorrichtung kann besonders kompakt ausgebildet werden und die Rotation des Plattenstapels ist über einen Elektromotor besonders leicht zu regeln, womit auch bei schwankenden Gasströmen schnell auf unterschiedlichen Bedarf an Flüssigkeit für die Gasbefeuchtung reagiert werden kann.

Ansonsten zeichnet die Vorrichtung sich im wesentlichen dadurch aus, dass die Einhaltung der Zielparameter durch eine einfach realisierbare Überwachung des Flüssigkeitspegels im Flüssigkeitsreservoir sowie der Temperatur und dem Druck in der Befeuchtungskammer erreicht werden kann zusammen mit einer Messung der Temperatur und gegebenenfalls relativen Feuchte eines einem Patienten zugeführten Atemgases.

Weitere Vorteile und Merkmale der Erfindung ergeben sich aus der nachfolgenden Beschreibung von Ausführungsbeispielen. Dabei zeigt
- Figur 1: die Prinzipskizze einer Vorrichtung zur Temperierung und Befeuchtung von Gas zum Beatmen eines Patienten mit einer Befeuchtung unter Überdruck;
- Figur 2: eine alternative Ausführung einer Vorrichtung zur Temperierung und Befeuchtung von Gas ohne einen Hochdruckbereich;
- Figur 3: eine weitere alternative Ausführungsform mit einer Bewegungsvorrichtung in Form von einem Plattenstapel.

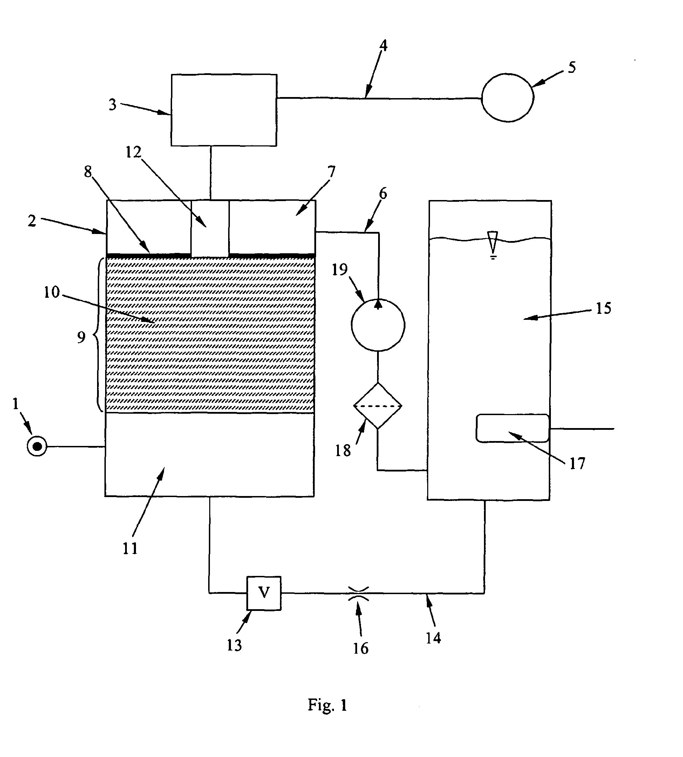

Figur 1 zeigt ein Ausführungsbeispiel einer erfindungsgemäßen Befeuchtüngsvorrichtung, in diesem Fall mit Positionierung vor dem Atemstromerzeugungsgerät.

Bei dem dargestellten Ausführungsbeispiel wird Atemgas einem Reservoir 1 entnommen, beispielsweise einer Druckgasflasche oder einer Druckgasleitung. Dieses üblicherweise sehr trockene Druckgas wird über einen Befeuchter 2 einem Atemstromerzeugungsgerät 3 zugeleitet. Hier wird das zugeführte Atemgas auf einen Druck entspannt (Atemgasniveau), der für eine Beatmung oder eine Atemtherapie benötigt wird und der etwas über dem Umgebungsdruck liegen kann. Das
Atemgas wird dann temperaturgeregelt über einen an dem Atemstromerzeugungsgerät angeschlossenen, beheizbaren Beatmungsschlauch 4 zu einem Patienten 5 geleitet.

Die Zuleitung vom Atemstromerzeugungsgerät 3 zum Patienten 5 erfolgt dabei bei sehr geringem kompressiblen Volumen des Atemsystems.

Wichtig ist, dass das dem Patienten zugeführte Atemgas exakt und konstant eine Sollfeuchte und
-temperatur aufweist, typischerweise nahe der Sättigungsgrenze, also fast 100% bei einer Temperatur von ca. 37 °C. Außerdem muss die Beatmung des Patienten unterbrechungsfrei durchzuführen sein.

Hierzu wird dem Befeuchter 2 über eine Leitung 6 beheizte Flüssigkeit zugeführt, die in diesem Ausführungsbeispiel Wasser von etwa 72 °C ist, die in dem Befeuchter 2 in eine Ringkammer 7 gelangt. Wenn im übrigen hier von Wasser die Rede ist, so sei noch einmal darauf hingewiesen, dass auch die Verwendung anderer geeigneter Flüssigkeiten möglich ist, die der Fachmann aufgrund seines Wissens auswählen kann. Auch können der Flüssigkeit bzw. im hier beschriebenen Ausführungsbeispiel dem Wasser Medikamente o.ä. zugesetzt werden, ohne dass dies im folgenden jeweils separat erwähnt würde.

Aus der Ringkammer 7 läuft das temperierte Wasser über einen Siebboden 8 in eine Berieselungskammer 9, die eine Füllung 10 enthält. Diese Füllung besteht in diesem Ausführungsbeispiel aus festen Strukturelementen mit großer Oberfläche und großen Aussparungen.

Während das Wasser aus der Ringkammer 7 über die große Oberfläche nach unten rieselt in die Bodenkammer 11 des Befeuchters 2, erwärmt und benetzt es die Füllung 10. Gleichzeitig strömt aus dem Reservoir 1 kommendes Atemgas in der Gegenrichtung durch die Füllung 10. Dabei erwärmt sich dieses Atemgas und nimmt gleichzeitig Feuchtigkeit auf, so dass es nahezu gesättigt über eine Sammelkammer 12 zum Atemstromerzeugungsgerät 3 geleitet werden kann.

Es sei an dieser Stelle auch darauf hingewiesen, dass der dem Befeuchter zugeführte Volumenstrom an beheiztem Wasser erheblich höher ist als der Volumenstrom, der in dem Befeuchter nur für die Sättigung des Atemgasstromes mit Feuchtigkeit benötigt würde. Damit wird erreicht, dass im Kontaktbereich von Gas und Flüssigkeit diese nicht signifikant auskühlt, wodurch die erforderliche Temperaturerhöhung des Atemgasstromes sichergestellt ist.

Im hier dargestellten Beispiel erfolgt der oben beschriebene Vorgang unter Druck, d.h. das Wasser und damit das Atemgas haben einen Druck von ca. 4,5 bar bei einer Temperatur von ca. 72 °C.

Durch die Entspannung des Atemgases von ca. 4,5 bar bei ca. 72 °C auf etwas über Umgebungsdruck und die Abkühlung auf eine Temperatur von ca. 37 °C behält das Atemgas seine relative Feuchte von nahezu 100 %.

Es sind im Befeuchter auch andere Temperatur-/Druckkombinationen möglich, solange gewährleistet ist, dass auch nach der Entspannung auf Atemdruckniveau das Atemgas bei der eingestellten Zieltemperatur die Zielfeuchte entsprechend den vordefinierten Parametern aufweist.

Wie bereits erklärt, wird im Befeuchter 2 die Flüssigkeit, die über die Füllung 10 gerieselt ist, in der Bodenkammer 11 gesammelt. Von hier fließt sie, gesteuert über ein Ventil 13, durch eine Rückflussleitung 14 in einen Vorratsbehälter 15. In der Rückflussleitung 14 ist dabei ein Druckminderer 16 vorgesehen, so dass der Vorratsbehälter 15 als solcher drucklos ist. Im hier dargestellten Ausführungsbeispiel ist der Druckminderer als Drossel ausgebildet.

Im Vorratsbehälter 15 wird über eine geregelte Heizung 17 die Wassertemperatur im hier vorliegenden Beispiel auf 72 °C gehalten, wobei eine derartige Temperaturregelung relativ einfach konstruktiv zu lösen ist. Das derart erwärmte Wasser wird über einen optionalen Filter 18 von einer Pumpe 19 durch die Leitung 6 wieder in die Ringkammer 7 des Befeuchters 2 gepumpt.

Der optionale Filter 18 gewährleistet hierbei, dass das umgewälzte Wasser frei von Partikeln und Mikroorganismen ist und somit auch das dem Atemstromerzeugungsgerät 3 zugeführte Atemgas im wesentlichen als keimfrei angesehen werden kann.

Durch die Pumpe 19 und das Ventil 13 bzw. den Druckminderer 16 kann der Vorratsbehälter 15 drucklos ausgebildet sein und kann jederzeit nachgefüllt werden.

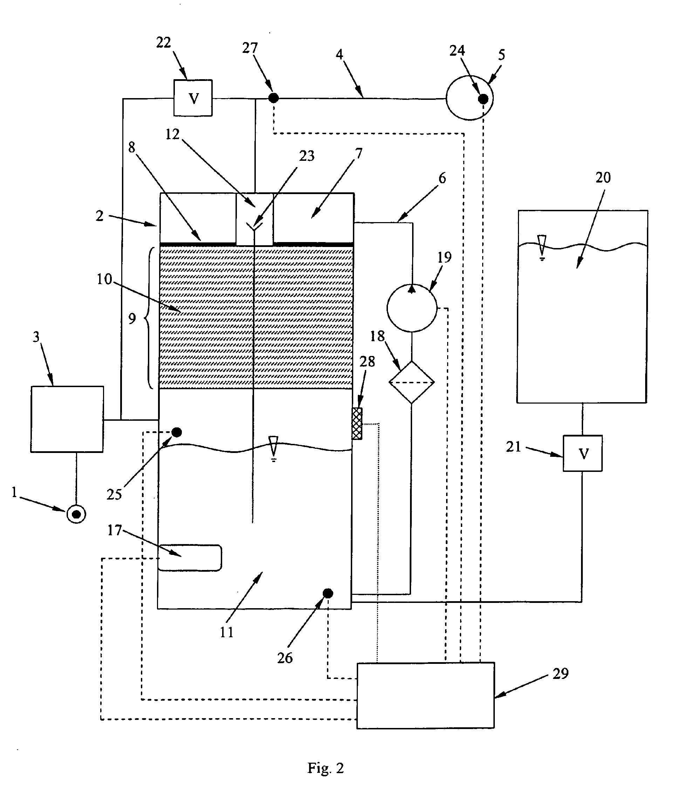

Ein alternatives Ausführungsbeispiel zeigt Figur 2. Hierbei ist der Befeuchter 2 wie bei den beschriebenen Verfahren nach dem Stand der Technik zwischen das Atemstromerzeugungsgerät 3 und den Patienten 5 geschaltet.

Um das komprimierbare Volumen und die Baugröße gering zu halten, ist die Vorrichtung für in der Beatmungstechnik übliche Gasströme (0 bis ca. 180 l/min) und auf normkonforme Befeuchtungsleistungen ausgelegt.

Dem Befeuchter 2 wird über eine Leitung 6 temperierte Flüssigkeit zugeführt, die in diesem Ausführungsbeispiel wiederum Wasser von etwa 37 °C ist, die in dem Befeuchter 2 in eine Verteilkammer 7 gelangt.

Aus der Verteilkammer 7 läuft das temperierte Wasser über einen Siebboden 8 in eine Berieselungskammer 9, die eine Füllung 10 enthält. Diese Füllung besteht in diesem Ausführungsbeispiel aus festen Strukturelementen mit großer Oberfläche und großen Aussparungen.

Im Sinne einer Vereinfachung der Vorrichtung ist denkbar, auf die Füllung 10 zu verzichten und die aus dem Siebboden 8 austretenden Flüssigkeitströpfchen einfach durch die Berieselungskammer 9 fallen zu lassen. Dies kann u.U. als vorteilhaft für die Reinigung und Aufbereitung angesehen werden, es ist aber zu berücksichtigen, dass bei gleichen Anforderungen an die Befeuchtungsleistung unter Umständen das Volumen der Berieselungskammer sowie die umgewälzte Flüssigkeitsmenge pro Zeit wesentlich zu vergrößern sind.

Während das Wasser aus der Verteilkammer 7 über die große Oberfläche nach unten rieselt in die Bodenkammer 11 des Befeuchters 2, erwärmt und benetzt es die Füllung 10. Gleichzeitig strömt aus dem Atemstromerzeugungsgerät 3 kommendes Atemgas in Gegenrichtung durch die Füllung 10. Dabei erwärmt sich dieses Atemgas und nimmt gleichzeitig Feuchtigkeit auf, so dass es nahezu gesättigt mit einer relativen Feuchte von etwa 95 - 100 % über die Sammelkammer 12 zum Patienten 5 geleitet werden kann.

Um Kondensatbildung zu vermeiden, wird vorzugsweise ein technologisch bekannter, geregelt beheizter Beatmungsschlauch 4 eingesetzt. Wichtig hierbei ist, dass keine Stelle der Wandung im Bereich des Gasstroms kälter als die Sättigungstemperatur der Gase ist, um Kondensation zu verhindern. Üblicherweise wird das Gas in diesem Beatmungsschlauch 4 noch etwas weiter erwärmt, z.B. auf 40 °C, um auch auf der letzten Strecke zum Patienten, insbesondere dem ungeheizten Tubus, eine Kondensation zu verhindern.

Es sei noch erwähnt, dass je nach Funktionsprinzip des jeweiligen Atemstromerzeugungsgerätes 3 nur ein einziger Beatmungsschlauch 4, oder mehrere eingesetzt werden, wobei hierbei ggf. auch die Ausatemluft des Patienten dem Atemstromerzeugungsgerät zugeführt werden kann. In diesen Fällen beziehen sich die o.g. Ausführungen zur Notwendigkeit bzw. zu den Möglichkeiten der Schlauchheizung auch auf alle weiteren Schläuche und Schlauchabschnitte, in denen die Kondensationsproblematik bestehen kann.

Wie bereits erklärt, wird im Befeuchter 2 die Flüssigkeit, die über die Füllung 10 gerieselt ist, in der Bodenkammer 11 gesammelt, die als Flüssigkeitsreservoir fungiert.

Hier wird die Flüssigkeit über eine geregelte Heizung 17 auf einer Wassertemperatur im hier vorliegenden Beispiel von 37 °C gehalten, wobei eine derartige Temperaturregelung relativ einfach konstruktiv zu lösen ist. Das derart erwärmte Wasser wird über einen optionalen Filter 18 von einer Pumpe 19 durch die Leitung 6 wieder in die Verteilkammer 7 des Befeuchters 2 gepumpt.

Der optionale Filter 18 gewährleistet hierbei, dass das umgewälzte Wasser frei von Partikeln und Mikroorganismen ist und somit auch das dem Patienten 5 zugeführte Atemgas im Wesentlichen als keimfrei angesehen werden kann. Alternativ oder ergänzend kann die Vorrichtung mit einer antimikrobiellen Oberfläche ausgestattet sein.

Über einen Flüssigkeits-Vorratsbehälter 20, z.B. eine Infusionsflasche, kann mittels eines Ventils 21 der Flüssigkeitspegel in der Bodenkammer 11 auf konstantem Niveau reguliert werden. Hierfür kann dieses Ventil insbesondere auch als Schwimmerventil ausgeführt sein. Wichtig ist hierbei, dass der hydrostatische Differenzdruck zwischen dem Flüssigkeits-Vorratsbehälter 20 und der Bodenkammer 11 stets größer ist als der höchstvorkommende Beatmungsdruck. Für die Praxis empfiehlt sich hieraus die Positionierung des Vorratsbehälters in mind. 1 m Höhe über der Bodenkammer.

Ein weiteres Ventil 22 dient als Bypassventil zwischen Gaseinlass und Gasauslass des Befeuchters 2. Dieses stellt sicher, dass bei einem zu hohen Differenzdruck, z.B. durch einen Defekt im Befeuchter, die Beatmung nicht behindert wird, und dient somit der Patientensicherheit.

Über eine in den Sammelbereich 12 integrierte Wasserfalle 23 wird überschüssiges und in der Luft mitgeführtes Wasser abgeschieden und in die Bodenkammer 11 zurückgeführt.

Zur Regelung des Befeuchtungsprozesses und zur Überwachung der Funktion steht das Regel-und Überwachungsgerät 29 mit dem Befeuchter 2 und dem Patienten 5 über verschiedene Sensoren, Steuer- und Regelleitungen in Verbindung. Über den Temperatursensor 24, welcher z.B. als handelsübliche Temperatursonde ausgeführt sein kann, wird die Patiententemperatur ermittelt. Diese kann in den Befeuchtungsprozess als Zielgröße eingehen. Alternativ kann diese Zieltemperatur auch durch die Kommunikation mit einem anderen Gerät, z.B. einem Überwachungsmonitor, vorgegeben werden. Über einen weiteren Temperatursensor 26 wird die Flüssigkeitstemperatur erfasst und dann mit dem Heizelement 17 geregelt.

Der dritte Temperatursensor 25 erfasst die Temperatur des eintretenden Gases. Bei einem zu hohen Anstieg des Flüssigkeitspegels in der Bodenkammer 11 infolge eines Versagens des Ventils 21 oder eines Ausfalls der Pumpe 19 kann ein Regel- und Überwachungsgerät 29 über die Temperaturänderung an diesem Sensor 25 den zu hohen Flüssigkeitspegel erkennen. In diesem Fehlerfall öffnet ein Bypassventil 22, woraufhin an einem Temperatursensor 27, der die Temperatur am Gasauslass erfasst, eine deutliche Temperaturabnahme zu messen ist. Dieser Sicherheitsmechanismus ist durch ein gezieltes Abschalten der Pumpe 19 und dem daraus folgenden Anstieg des Flüssigkeitspegels und überprüfbar.

Über einen fest mit dem Befeuchter 2 verbundenen Transponder 28 steht das Regel- und Überwachungsgerät 29 mit dem Befeuchter 2 in Verbindung, um ihn zu identifizieren und hinsichtlich seiner Lebensdauer zu überwachen.

In dem Regel- und Überwachungsgerät 29 kann auch die Leistungsaufnahme der Pumpe 19 überwacht werden. Da die Leistungsaufnahme in Zusammenhang mit der Höhe des Flüssigkeitspegels in der Bodenkammer 11 steht, kann damit auch ein zu niedriger Flüssigkeitspegel, z.B. bedingt durch einen leeren Vorratsbehälter 20 oder ein defektes Ventil 21, oder eine mangelhafte Flüssigkeitsumwälzung, z.B. durch eine Verstopfung des Siebbodens 8, erkannt werden.

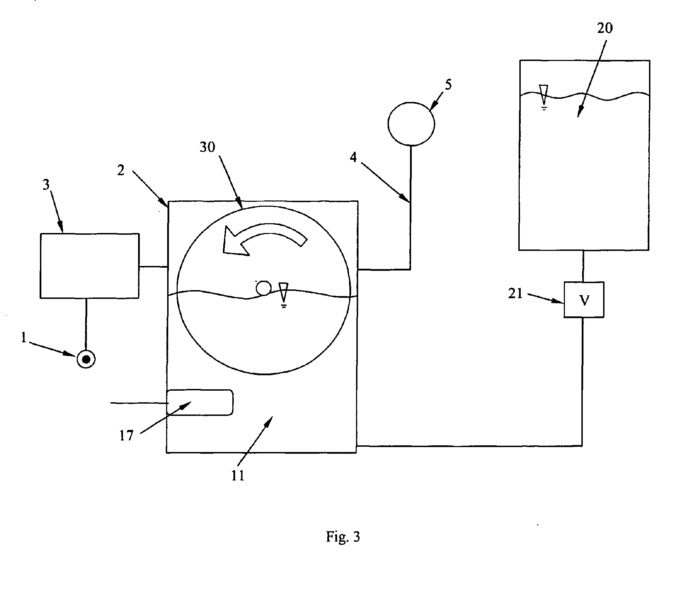

Alternativ wäre auch eine Anordnung gemäß Figur 3 denkbar:

Hierbei befindet sich der Befeuchter 2 entsprechend Figur 2 zwischen Atemstromerzeugungsgerät 3 und Patient 5. Die Flüssigkeit befindet sich in einer Bodenkammer 11 und wird über die Heizung 17 auf konstante Temperatur geregelt.

Der Flüssigkeitspegel in der Bodenkammer 11 wird entsprechend Figur 2 mittels Vorratsbehälter 20 und Ventil 21 auf konstantem Niveau gehalten.

Feste Strukturelemente des Austauschelementes 30 tauchen zyklisch, insbesondere mittels Rotation, zumindest teilweise so in die Flüssigkeit ein, dass dadurch ein ausreichend häufiger Austausch des Wassers im Kontaktbereich erfolgt. Hierbei lässt sich die Menge der beim Eintauchen mitgenommenen Flüssigkeit durch die geometrische Gestaltung der Strukturelemente wesentlich beeinflussen, z.B. durch radiale Rippen o.ä. Weiterhin kann durch eine hohe Rotationsgeschwindigkeit der Energietransport vergrößert werden.

Bei den beschriebenen Ausführungen z.B. gemäß den Figuren 1, 2 und 3 sowie korrekter Dimensionierung kann ein stark schwankender und unterbrochener Gasstrom im Wesentlichen auf konstante Temperatur und Sättigung gebracht werden, ohne z.B. die Gefahr der Überhitzung des Gases nach Unterbrechung oder einer zu geringer Feuchte bei plötzlichem hohem Gasstrom.

Für die erfindungsgemäße Lösung der Aufgabe ist es hierzu erforderlich, den Kontaktbereich von Flüssigkeit und Gas so zu dimensionieren und mit einer derartigen geometrischen Struktur zu versehen, dass auch beim maximal in der Anwendung vorgesehenen Spitzen-Gasstrom der Soll-Stoff- und Energieaustausch nahezu vollständig stattfinden kann. Diese Dimensionierung kann geprüft werden, indem der maximal vorgesehene Spitzen-Gasstrom konstant durch die erfindungsgemäße Vorrichtung geleitet wird. Bei korrekter Dimensionierung entspricht die Gastemperatur nach Durchströmen der Befeuchtungskammer nahezu der Flüssigkeitstemperatur vor deren Eintritt in die Befeuchtungskammer.

Das Mitreißen von Wassertröpfchen im Gasstrom wird durch geeignete Maßnahmen verhindert (z.B. Vergrößerung des Strömungs-Querschnitts).

Durch den Anschluss von beheizten Schläuchen für den weiteren Transport des Gases kann die Kondensatbildung im Schlauch wirkungsvoll verhindert werden.

Sofern eine relative Feuchte von weniger als 100 % gewünscht ist, so lässt sich dies über die Nachschaltung eines geeigneten Heizelementes für das befeuchtete Gas realisieren: Das Gas wird im erfindungsgemäßen Befeuchter auf diejenige Temperatur und Sättigung gebracht, deren Absolutgehalt an Feuchte den Anforderungen entspricht. Über eine nachgeschaltete Gasheizung oder durch Mischung mit trockenem Gas wird das den Befeuchter verlassende Gas nun auf die eigentliche Zieltemperatur und relative Zielfeuchte gebracht. Dadurch ist die Erzeugung verschiedener Gastemperaturen und -feuchten realisierbar.

Die erfindungsgemäße Vorrichtung ist nicht nur für die Beatmungstechnik geeignet, sondern für alle Anwendungen, in denen ein variabler Gasstrom von Partikeln zu reinigen und/oder auf konstante Temperatur und Feuchte zu bringen ist. Beispiele hierfür sind z.B. die Insufflation von Gasen in Körperhöhlen (z.B. CO₂ bei der Laparoskopie), die Bereitstellung von Atemgasen für Atemschutz-Anwendungen (z.B. Lackierung), Inhalationsanwendungen aller Art, die Klimatisierung von Räumen (z.B. Gebäude, Fahrzeuge, Flugzeuge, ...) entweder alleine oder in Kombination mit Klima-Anlagen, etc.

## Patentansprüche

1. Vorrichtung zur Temperierung und Befeuchtung von Gas,
mit
- einem Flüssigkeitsreservoir (11, 15, 20),
- einer Befeuchtungskammer (9), die einen Zufluss und einen Abfluss für das Gas aufweist,
- einer Bewegungsvorrichtung (19, 30), die dazu angepasst ist, um die Flüssigkeit durch das Gas zu bewegen, mit
einer geregelten Heizung (17), die dazu angepasst ist, die Flüssigkeit zu beheizen.

2. Vorrichtung gemäß Anspruch 1,
wobei
die Befeuchtungskammer (9) als Berieselungskammer ausgebildet ist.

3. Vorrichtung gemäß Anspruch 2,
wobei
in der Berieselungskammer (9) eine Füllung (10) vorgesehen ist, die eine große Oberfläche aufweist.

4. Vorrichtung gemäß Anspruch 1,
wobei
die Befeuchtungskammer (9) unter Überdruck steht.

5. Vorrichtung gemäß Anspruch 1,
wobei
die Bewegungsvorrichtung eine Pumpe (19) ist, die der Berieselungskammer (9) die Flüssigkeit aus dem Flüssigkeitsreservoir (11, 15) zuführt, wobei Berieselungskammer (9) und Flüssißkeits-reservoir (11, 15) in einem Kreislauf verbunden sind.

6. Vorrichtung gemäß Anspruch 1,
wobei
die Bewegungsvorrichtung ein in die Flüssigkeit in dem Flüssigkeitsreservoir (11) teilweise eintauchender rotierender Körper (30) ist.

7. Vorrichtung gemäß Anspruch 6,
wobei
der Körper (30) ein Stapel zu einander beabstandeter Platten ist.

8. Verfahren zur Temperierung und Befeuchtung eines Gases für die Bereitstellung von Atemgas für Atemschutz = anwendungen oder bei der Klimatisierung von Räumen mit einer Vorrichtung nach einem oder mehreren der Ansprüche 1-7 enthaltend die Schritte:
Zuführen eines Gases aus einem Reservoir (1),
geregelte Erzeugung eines Gasstromes durch eine Befeuchtungskammer (9),
Erwärmung und Befeuchtung des Gases **dadurch**, daß eine geregelte Heizung (17) für eine Flüssigkeit vorgesehen wird, welche mittels einer Bewegungsvorrichtung (19, 30) durch das Gas bewegt wird, wobei die Befeuchtung mittels Berieselung erfolgt, und Zuleiten des Gases zu einem Verbraucher.

9. Verfahren gemäß Anspruch 8,
wobei
die Befeuchtung bei einem Überdruck erfolgt, vorzugsweise bei einem Druck von ca. 4,5 bar, und bei einer Temperatur, die gegenüber einer Zuführungstemperatur zu dem Verbraucher erhöht ist, vorzugsweise bei einer Temperatur von ca. 72 °C, wobei das Atemgas auf Atemgasniveaü entspannt wird, vorzugsweise maximal 0,1 bar über Umgebungsdruck, bei einer Temperatur von vorzugsweise ca. 37 °C und einen vorgewählten relativen Feuchte von vorzugsweise ca. 100 %. für die Bereitstellung von Atemgas für Atemschutzanwendungen oder bei der Klimatisierung von Räuman mit einer Vorrichtung nach einem oder mehreren der Ansprüche 1-7.

## Claims

1. A device to heat and humidify gas comprising:
- a fluid reservoir (11, 15, 20),
- a humidification chamber (9) having an inlet and an outlet for the gas,
- a drive device (19, 30) adapted to move fluid through the gas,
with a controlled heater device (17) adapted for heating the fluid.

2. A device according to claim 1
wherein said humidification chamber (9) is a sprinkling type chamber.

3. A device according to claim 2,
wherein a filling (10) is provided in said humidification chamber (9), said filling forming a large surface.

4. A device according to claim 1,
wherein said humidification chamber (9) is pressurized.

5. A device according to claim 1,
wherein said drive device is a pump (19) feeding fluid from the fluid reservoir (11, 15) to the sprinkling type chamber (9), the sprinkling type chamber (9) and the fluid supply reservoir (11, 15) being connected in a circuit.

6. A device according to claim 1,
wherein said drive device is a rotating body (30) partially dipping into the fluid within the fluid reservoir (11).

7. A device according to claim 6,
wherein the body (30) is a stack of plates connected to each other with a distance.

8. A process for heating and humidification of a gas to provide respiratory gas in breathing protection applications or for air conditioning of rooms with a device according to one or a plurality of claims 1 to 7 comprising the steps:
- supplying of a gas from a reservoir (1),
controlled generating of a gas flow, through a humidification chamber (9),
- heating and humidifying of said gas, in that a controlled heater device (17) is provided for a fluid that is moved through the gas with a drive device (19, 30), wherein the humidification is effected by sprinkling, and
- feeding the gas to a consumer;

9. A procedure according to claim 8,
wherein the humidification takes place under pressure, preferably with a pressure of about 4.5 bar, and at a temperature higher than the temperature of the gas flow directed to the consumer, preferably at a temperature of about 72 °C, while the respiratory gas is depressurized to the level of normal respiration, preferably maximal 0.1 bar above the pressure of ambient air, with a temperature of preferably about 37 °C und a preselected relative humidity of preferably about 100 %.

## Revendications

1. Dispositif d'équilibrage de la température et d'humidification de gaz, avec
- un réservoir de liquide (11, 15, 20),
- une chambre d'humidification (9), qui présente une admission et une évacuation pour le gaz,
- un dispositif déplaceur (19, 30), qui est adapté pour déplacer le liquide à travers le gaz, avec
un chauffage régulé (17) qui est adapté pour chauffer le liquide.

2. Dispositif selon la revendication 1, sachant que la chambre d'humidification (9) est réalisée sous forme de chambre d'arrosage.

3. Dispositif selon la revendication 2, sachant qu'un matériau de remplissage (10), qui présente une grande surface, est prévu dans la chambre d'arrosage (9).

4. Dispositif selon la revendication 1, sachant que la chambre d'humidification (9) est soumise à une surpression.

5. Dispositif selon la revendication 1, sachant que le dispositif déplaceur est une pompe (19) qui apporte à la chambre d'humidification (9) le liquide provenant du réservoir de liquide (11, 15), sachant que la chambre d'humidification (9) et le réservoir de liquide (11, 15) sont reliés en circuit fermé.

6. Dispositif selon la revendication 1, sachant que le dispositif déplaceur est un corps rotatif (30) s'enfonçant partiellement dans le liquide dans le réservoir de liquide (11).

7. Dispositif selon la revendication 6, sachant que le corps (30) est une pile de plaques distantes les unes des autres.

8. Procédé d'équilibrage de la température et d'humidification d'un gaz, pour la préparation de gaz respiratoire pour des applications de protection respiratoire ou pour la climatisation de locaux, au moyen d'un dispositif selon une ou plusieurs des revendications 1 à 7, comprenant les étapes suivantes :
alimentation d'un gaz à partir d'un réservoir (1),
production régulée d'un flux de gaz à travers une chambre d'humidification (9), chauffage et humidification du gaz par le fait qu'un chauffage régulé (17) est prévu pour un liquide qui, au moyen d'un dispositif déplaceur (19, 30), est déplacé à travers le gaz, sachant que l'humidification s'effectue par arrosage, et
apport du gaz à un utilisateur.

9. Procédé selon la revendication 8, sachant que l'humidification s'effectue en présence d'une surpression, de préférence à une pression d'environ 4,5 bars, et à une température qui est accrue par rapport à une température d'apport à l'utilisateur, de préférence à une température d'environ 72 °C, sachant que le gaz respiratoire est détendu à un niveau de gaz respiratoire, de préférence au maximum à 0,1 bar au-dessus de la pression ambiante, avec une température d'environ 37 °C de préférence et une humidité relative présélectionnée d'environ 100 % de préférence.
